# EUROPEAN PATENT SPECIFICATION

(11) **EP 0 444 492 B1**
(45) Date of publication and mention of the grant of the patent: **10.01.1996**
(21) Application number: 91102240.8
(22) Date of filing: 18.02.1991
(51) Int. Cl.: A61K 31/725

(54) **Topically administered compositions based on high molecular weight hyaluronic acid for treating inflammations of the oral cavity, and for oral cavity hygiene and cosmetic treatment**
Topisch anwendbare Zusammensetzungen auf der Basis von hoch molekularer Hyaluronsäure zur Behandlung von Entzündungen der Mundhöhle, zu deren Hygiene und kosmetischer Behandlung
Compositions pour l'administration topique à base d'acide hyaluronique de haut poids moléculaire pour traiter les inflammations de la cavité buccale, pour l'hygiène de la cavité buccale et comme traitement cosmétique

(30) Priority: 21.02.1990 IT 1943890
(43) Date of publication of application: 04.09.1991
(73) Proprietor: RICERFARMA Srl, I-20144 Milano (IT)
(72) Inventor: Di Schiena, Michele Giuseppe, I-20080 Cisliano (Milan) (IT)
(74) Representative: Gervasi, Gemma, Dr.

(56) References cited:
- EP-A- 0 138 572
- EP-A- 0 197 718
- EP-A- 0 243 867
- WO-A-84/04453
- WO-A-88/07060
- PHARMACOMETRICS, vol. 28, no. 6, 1984, pages 1123-1135; K. MIYAZAKI et al.: "Studies on analgesic and anti-inflammatory effects of sodium hyaluronate (SPH)"
- PATENT ABSTRACTS OF JAPAN, vol. 7, no. 118 (C-167)[1263], 21st May 1983; & JP-A-58 37 001
- CHEMICAL ABSTRACTS, vol. 99, no. 26, 26th December 1983, page 382, abstract no. 218566e, Columbus, Ohio, US; & JP-A- 58 84 801
- CHEMICAL ABSTRACTS, vol. 99, no. 16, 17th October 1983, page 356, abstract no. 128315t, Columbus, Ohio, US; & JP-A- 58 37 001
- MIN. STOM., vol. 17, 1968, pages 140-156; F. BRANDIMARTE: "Acido ialuronico e parodontopatie"

## Description

This invention relates to the use of the sodium salt of high molecular weight hyaluronic acid as active principle in the preparation of pharmaceutical compositions for topical application for the therapy and prophylaxis of inflammatory affections of the oral cavity, and for hygienic and cosmetic treatment of the oral cavity.

Hyaluronic acid is the most important asulphurated mucopolysaccharide acid of the fundamental matter of the connective tissue, both from the strictly biochemical and from the physiological viewpoints.

In man, hyaluronic acid is present not only in the connective tissue but also in the important biological fluids such as vitreous humor, aqueous humor, etc. and in the umbilical cord.

It has practically zero toxicity, and specific contraindications for human use are not known.

As in the case of other components of natural substances, hyaluronic acid can be obtained by extraction from the relative natural substances, for example it can be extracted from chicken crests, or can be produced biotechnologically.

Hyaluronic acid has a very wide range of molecular weights, which can vary from 30,000 to more than 15,000,000, depending on the type of process used.

Said acid has been used in the form of its sodium salt for some time, both in human therapy and in cosmetic treatment.

In this respect the exogenous application of hyaluronic acid has a beneficial effect in favouring the connective organization, and in addition it opposes the inflammatory process induced by hyaluronidase-producing germs; it facilitates resolution of the phlogistic component, reduces abnormal capillary permeability, accelerates tissue repair processes and it performs an antiedematogenic action by metabolically binding free water to its molecular structures.

The therapeutic indications for hyaluronic acid are numerous, including abrasive-excoriative dermatopathies, ulcers deriving from arteriosclerotic vasculopathies, varicose ulcers, cicatrization delays and surgical excisions.

In cosmetic practice hyaluronic acid is used for its restorative, tonicizing, dermo-reparation, cicatrizing and hydrating properties. For example in USP 4,736,024 hyaluronic acid or a salt thereof is used as a vehicle for pharmacologically active substances in pharmaceutical compositions which can be topically administered.

This patent describes the use of hyaluronic acid in pharmaceutical compositions for application to the oral cavity, however in this patent the hyaluronic acid is used only as a vehicle which allows faster absorption of the topically applied active principle. Moreover, the compositions amply described and claimed in it are for ophthalmic and dermatological treatment.

It is therefore probable that prolonged therapeutic use in the oral cavity of hyaluronic acid associated with medicaments presents risks due to the possible side effects of the medicament on the gingival tissue and risks connected with the inevitable ingestion of the medicament itself.

The compositions described in this patent can hardly be used for practical preventive hygiene.

Hyaluronic acid has been used as active principle with success in the therapy of different periodontopathies by injecting it submucously directly into the gingiva (F. Brandimarte, Min. Stom. 17, 140-156, 1968).

This type of hyaluronic acid administration is strictly the competence of the specialist medical practitioner and in addition there are infiltration-related risks, and moreover it cannot be used extensively and it is unpleasant for the patient.

Furthermore, such administration cannot be used for the more common inflammatory pathologies of the gingiva, these being the most common.

EP-A3-243867 teaches the treatment of traumatized and inflamed mammalian tissues by administering hyaluronic acid or its salts by any of the accepted routes of administration (such as intramuscular, intravenous, subcutaneous injection, or topical application) except direct application to the affected tissues, hyaluronic acid being effectively transported to the site of action by the body's internal processes.

With regard to topical compositions, EP-A3-243867 teaches that sodium hyaluronate is effective only when administered in combination with transdermal carriers, such as methyl salicylate, sodium salicylate, benzyl alcohol, oleic acid, propylene glycol, sodium glycolate, polyoxyethylene-10-cethyl ether, sodium EDTA, sodium dodecyl sulphate or dimethyl sulphoxide.

It has now been surprisingly found that hyaluronic acid in sodium salt form and characterised by a molecular weight of between 800,000 and 4,000,000, and preferably between 1,000,000 and 2,000,000, can be used as active principle in the preparation of pharmaceutical compositions for topical administration in the treatment and prophylaxis of inflammatory affections of the oral cavity and for cosmetic treatment and hygiene of the oral cavity, on condition that said compositions do not contain any compound selected from the group consisting of methyl salicylate, sodium salicylate, benzyl alcohol, oleic acid, propylene glycol, sodium glycolate, polyoxyethylene-10-cethyl ether, sodium ethyelenediaminetetracetic acid (EDTA), sodium dodecyl sulphate and dimethyl sulphoxide.

Consequently the present invention provides topically administered pharmaceutical compositions for the prophylaxis and treatment of the oral cavity and for oral cavity cosmetic treatment and relative hygiene, containing as active principle hyaluronic acid characterised by an average molecular weight of between 800,000 and 4,000,000, and preferably between 1,000,000 and 2,000,000 on condition that said compositions do not contain any compound selected from the group consisting of methyl salicylate, sodium salicylate, benzyl alcohol, oleic acid, propylene glycol, sodium glycolate, polyoxyethylene-10-cethyl ether, sodium ethyelenediaminetetracetic acid (EDTA), sodium dodecyl sulphate and dimethyl sulphoxide.

The compositions according to the invention generally contain from 0.005% to 10% of Sodium Hyaluronate.

The compositions according to the present invention for therapeutic use generally contain between 0.2 and 10% by weight, based on the total composition weight, and preferably between 0.2 and 1% by weight of said active principle.

The compositions according to the present invention for oral cavity prophylactic, cosmetic and hygienic use contain between 0.005 and 0.1% by weight, based on the total composition weight, of said active principle. The latest preferably contain 0.01% by weight of sodium hyaluronate.

The compositions according to the present invention are administered topically and can be in the form of gingival pastes, toothpastes, mouthwashes and adhesive pastes and powders.

The pharmaceutical compositions according to the present invention generally contain conventional excipients, for example polyalcohols such as sorbitol and/or maltitol, glycols such as polyethyleneglycol, thickeners such as carboxymethylcellulose, preservatives such as methyl or propyl paraoxybenzoate, flavouring agents such as peppermint, sweeteners such as saccharin, and colouring agents.

The compositions according to the present invention for therapeutic use are advantageously used for gingival affections, characterised by inflammatory manifestations of the gingival tissue, such as gingivitis, stomatitis, irritations due to mechanical causes such as fixed or mobile prostheses or surgical operations etc.

The gingival pastes according to the present invention can also be used during the dentition stage in children.

The following examples are given for the purpose of illustrating the present invention but without implying any limitation thereon.

### EXAMPLE 1 Gingival paste in gel form

5 g of sodium hyaluronate of molecular weight 1,000,000 are carefully dispersed in 70 ml of water containing a preservative. 25 g of 70% sorbitol are added and the mixture is carefully mixed.

### EXAMPLE 2 Gingival paste in gel form

54 g of sodium carboxymethylcellulose of medium viscosity type are dispersed in 774 g of water (containing 0.13% of p-oxybenzoate and 0.007% of propyl p-oxybenzoate as preservatives).

240 g of an aqueous solution containing 1% of sodium hyaluronate (molecular weight 1,500,000) are added followed by 120 g of 70% sorbitol.

The system is stirred until a homogeneous transparent mass is obtained, which is then flavoured with 6 g of peppermint.

### EXAMPLE 3 Gingival paste in gel form

Example 2 is repeated but using a 70% aqueous maltitol solution instead of the 70% sorbitol.

### EXAMPLE 4 Toothpaste

A toothpaste is prepared by mixing the following components in the indicated proportions, which are by weight:

| | |
|---|---|
| amorphous silica | 18.00% |
| sorbitol | 74.5% |
| sodium carboxymethylcellulose | 0.2% |
| sodium hyaluronate (average M.W. 1,500,000) | 0.1% |
| saccharin | 0.20% |
| 50% aqueous NaOH | 0.30% |
| lauryl sulphate | 1.50% |
| sodium monofluorosulphate | 0.85% |
| water | 3 % |
| flavouring agents | 1 % |

colouring agents as required

### EXAMPLE 5 Mouthwash

The mouthwash is prepared by mixing the following components in the indicated proportions.

| | |
|---|---|
| Deionized water FU | 98 g |
| sodium hyaluronate (average M.W. 1,500,000) | 0.01% |

preservative as required
flavouring agents as required

### EXAMPLE 6 Adhesive powder for dentures

An adhesive powder for dentures is prepared by mixing the components in the following proportions:

| | |
|---|---|
| high viscosity sodium carboxymethylcellulose | 70% |
| sodium hyaluronate (average M.W. 2,000,000) | 1% |
| polyethoxylated polymer | 28% |
| peppermint flavour | 1% |

### EXAMPLE 7 Adhesive paste for dentures

An adhesive paste for dentures is prepared by mixing the following components in the indicated proportions:

| | |
|---|---|
| sodium hyaluronate (average M.W. 1,500,000) | 0.2% |
| lidocaine | 4 % |
| phenol | 0.1% |
| white vaselin | 57.7% |
| high viscosity sodium carboxymethylcellulose | 22 % |
| liquid paraffin | 8 % |
| cholesterol | 2 % |
| stearyl alcohol | 3 % |
| anhydrous alcohol | 3 % |

The gingival paste prepared in Example 2 containing 0.2% of sodium hyaluronate of average molecular weight 1,500,000 was tested on 10 patients.

The chosen patients showed various degrees of parodontal pathology. The first group comprising 6 patients suffered from simple marginal gingivitis, whereas the second group comprising the remaining 4 patients had undergone parodontal surgery.

With the first group it was noted that the topical use of the gingival paste containing hyaluronic acid resulted always by the second day in a reduction in symptomatology, characterised essentially by hypersensitivity to heat stimuli and slight bleeding on brushing, with complete recovery within one week.

With the second group examined, recovery was slower due essentially to the fact that a surgical wound was present which alone required about one week for recovery.

For this reason the compound was applied only during the latter stages of recovery mainly because the wound was protected by a parodontal compress. A clear improvement in condition was also noted in this group, to the extent that on termination of treatment the mucosa at the wound level was trophic and pink-coloured.

In conclusion the product was of considerable help in recovery, in that with the first group it participated in normal oral hygiene and with the second group it facilitated the normal physiological reparation processes.

Tests were conducted on the same patients with other compositions deriving from the aforesaid examples, the results obtained being comparable with those described.

## Claims (Claims for the following Contracting State(s): DE, FR, GB, IT)

1. Use of hyaluronic acid in the form of its sodium salt, characterised by an average molecular weight of between 800,000 and 4,000,000, as active principle in the preparation of pharmaceutical compositions for topical application both for the therapy and prophylaxis of inflammatory affections of the oral cavity, and for oral cavity hygiene and cosmetic treatment, on condition that said compositions do not contain any compound selected from the group consisting of methyl salicylate, sodium salicylate, benzyl alcohol, oleic acid, propylene glycol, sodium glycolate, polyoxyethylen-10-cethyl ether, sodium-ethylenediaminetetraacetic acid (EDTA), sodium dodecylsulphate and dimethylsulphoxide (DMSO).

2. The use of hyaluronic acid in the form of its sodium salt as claimed in claim 1, characterised in that the average molecular weight of the hyaluronic acid is between 1,000,000 and 2,000,000.

3. Topical compositions containing the sodium salt of hyaluronic acid as the sole active principle in accordance with one of the preceding claims in combination with excipients, for the therapeutic and prophylactic treatment of inflammatory affections of the oral cavity, and for oral cavity hygiene and cosmetic treatment, on condition that said compositions do not contain any compound selected from the group consisting of methyl salicylate, sodium salicylate, benzyl alcohol, oleic acid, propylene glycol, sodium glycolate, polyoxyethylen-10-cethyl ether, sodium-ethylenediaminetetraacetic acid (EDTA), sodium dodecylsulphate and dimethylsulphoxide (DMSO).

4. The topical compositions as claimed in claim 3, containing the active principle in amounts ranging from 0.005 to 10% by weight based on the total composition weight.

5. The topical compositions for the therapeutic treatment of inflammatory affections of the oral cavity as claimed in claim 4, containing the active principle in amounts ranging from 0.2 and 10% by weight based on the total composition weight.

6. The topical compositions for the therapeutic treatment of inflammatory affections of the oral cavity as claimed in claim 5, containing the active principle in amounts ranging from 0.2 and 1% by weight based on the total composition weight.

7. The topical compositions for the prophylaxis, cosmetic treatment and hygiene of the oral cavity as claimed in claim 4, containing the active principle in a concentration of between 0.005 and 0.1% by weight based on the total composition weight.

8. The topical compositions as claimed in claim 7, containing the active principle in a concentration of 0.01% by weight, based on the total composition weight.

9. The compositions as claimed in claim 3, in the form of gingival pastes, toothpastes, mouthwashes, adhesive pastes and powders.

10. The topical compositions as claimed in one of claims 5 and 6 for the treatment of gingivitis, stomatitis and irritation due to mechanical causes or surgery.

11. Gingival pastes as claimed in claim 9 for the dentition stage in children.

## Claims (Claims for the following Contracting State(s): ES, GR)

1. A process for the preparation of compositions for topical application both for the therapy and prophylaxis of inflammatory affections of the oral cavity, and for oral cavity hygiene and cosmetic treatment, containing the sodium salt of hyaluronic acid of average molecular weight of between 800,000 and 4,000,000 as the sole active principle, in combination with excipients, on condition that said compositions do not contain any compound selected from the group consisting of methyl salicylate, sodium salicylate, benzyl alcohol, oleic acid, propylene glycol, sodium glycolate, polyoxyethylen-10-cethyl ether, sodium-ethylenediaminetetraacetic acid (EDTA), sodium dodecylsulphate and dimethylsulphoxide (DMSO), said process comprising admixing the active principle with the excipients.

2. The process as claimed in claim 1, characterised in that the average molecular weight of the hyaluronic acid is between 1,000,000 and 2,000,000.

3. The process as claimed in claim 1, characterised in that the active principle is in amounts ranging from 0.005 to 10% by weight based on the total composition weight.

4. The process as claimed in claim 1, characterised in that said compositions for the therapy of inflammatory affections of the oral cavity contain the active principle in amounts ranging from 0.2 and 10% by weight based on the total composition weight.

5. The process as claimed in claim 4, characterised in that said compositions for the therapy of inflammatory affections of the oral cavity contain the active principle in amounts ranging from 0.2 and 1% by weight based on the total composition weight.

6. The process as claimed in claim 3, characterised in that said compositions for prophylaxis of inflammatory affections, cosmetic treatment and hygiene of the oral cavity contains the active principle in a concentration of between 0.005 and 0.1% by weight based on the total composition weight.

7. The process as claimed in claim 6, characterised in that said compositions contain the active principle in a concentration of 0.01% by weight, based on the total composition weight.

8. The process as claimed in claim 1, characterised in that said compositions are in the form of gingival pastes, toothpastes, mouthwashes, adhesive pastes and powders.

9. The process as claimed in claim 4 or 5, characterised in that said compositions are useful for the treatment of gingivitis, stomatitis and irritation due to mechanical causes or surgery.

10. The process as claimed in claim 8, characterised in that said gingival pastes are useful during the dentition stage in children.

## Patentansprüche (Patentansprüche für folgende(n) Vertragsstaat(en): DE, FR, GB, IT)

1. Verwendung der Hyaluronsäure in der Form ihres Natriumsalzes, gekennzeichnet durch ein mittleres Molekulargewicht zwischen 800.000 und 4.000.000, als Wirkstoff bei der Herstellung von pharmazeutischen Zusammensetzungen für eine topische Verabreichung sowohl für die Therapie und Prophylaxe von entzündlichen Erkrankungen der Mundhöhle als auch für die Hygiene der Mundhöhle und eine kosmetische Behandlung, unter der Bedingung, daß die genannten Zusammensetzungen keine Verbindung enthalten, die aus der Gruppe ausgewählt ist, welche aus Methylsalicylat, Natriumsalicylat, Benzylalkohol, Ölsäure, Propylenglycol, Natriumglycolat, Polyoxyethylen-10-cetylether, Natriumsalz der Ethylendiaminotetraessigsäure (EDTA), Natriumdodecylsulfat oder Dimethylsulfoxid (DMSO) besteht.

2. Die Verwendung der Hyaluronsäure in der Form ihres Natriumsalzes, wie sie im Anspruch 1 beansprucht wird, dadurch gekennzeichnet, daß das mittlere Molekulargewicht der Hyaluronsäure zwischen 1.000.000 und 2.000.000 liegt.

3. Topische Zusammensetzungen, welche das Natriumsalz der Hyaluronsäure als alleinigen Wirkstoff gemäß einem der vorhergehenden Ansprüche in Kombination mit Vehikeln enthalten, für die therapeutische und prophylaktische Behandlung von entzündlichen Erkrankungen der Mundhöhle, und für die Hygiene der Mundhöhle und die kosmetische Behandlung, unter der Bedingung, daß die genannten Zusammensetzungen keine Verbindung enthalten, die aus der Gruppe ausgewählt ist, welche aus Methylsalicylat, Natriumsalicylat, Benzylalkohol, Ölsäure, Propylenglycol, Natriumglycolat, Polyoxyethylen-10-cetylether, Natriumsalz der Ethylendiaminotetraessigsäure (EDTA), Natriumdodecylsulfat und Dimethylsulfoxid (DMSO) besteht.

4. Die topischen Zusammensetzungen, wie sie im Anspruch 3 beansprucht sind, welche den Wirkstoff mit einem Gehalt im Bereich von 0,005 bis 10 Gew.%, bezogen auf das Gesamtgewicht der Zusammensetzung, enthalten.

5. Die topischen Zusammensetzungen für die therapeutische Behandlung von entzündlichen Erkrankungen der Mundhöhle, wie sie im Anspruch 4 beansprucht werden, welche den Wirkstoff mit einem Gehalt im Bereich von 0,2 bis 10 Gew.%, bezogen auf das Gesamtgewicht der Zusammensetzung, enthalten.

6. Die topischen Zusammensetzungen für die therapeutische Behandlung von entzündlichen Erkrankungen der Mundhöhle, wie sie im Anspruch 5 beansprucht werden, welche den Wirkstoff mit einem Gehalt im Bereich von 0,2 bis 1 Gew.%, bezogen auf das Gesamtgewicht der Zusammensetzung, enthalten.

7. Die topischen Zusammensetzungen für die Prophylaxe, kosmetische Behandlung und Hygiene der Mundhöhle, wie sie im Anspruch 4 beansprucht werden, welche den Wirkstoff in einer Konzentration zwischen 0,005 und 0,1 Gew.%, bezogen auf das Gesamtgewicht der Zusammensetzung, enthalten.

8. Die topischen Zusammensetzungen, wie sie im Anspruch 7 beansprucht werden, welche den Wirkstoff in einer Konzentration 0,01 Gew.%, bezogen auf das Gesamtgewicht der Zusammensetzung, enthalten.

9. Die Zusammensetzungen, wie sie im Anspruch 3 beansprucht werden, in der Form von Zahnfleischpasten, Zahnpasten, Mundwässern, Haftpasten und Haftpulvern.

10. Die topischen Zusammensetzungen, wie sie in einem der Ansprüche 5 und 6 beansprucht werden, für die Behandlung von Gingivitis, Stomatitis, von Reizungen aufgrund mechanischer Ursachen oder aufgrund von Operationen.

11. Zahnfleischpasten, wie sie im Anspruch 9 beansprucht werden, für die Zahnung bei Kindern.

## Patentansprüche (Patentansprüche für folgende(n) Vertragsstaat(en): ES, GR)

1. Ein Verfahren zur Herstellung von Zusammensetzungen für eine topische Verabreichung sowohl für die Therapie und Prophylaxe von entzündlichen Erkrankungen der Mundhöhle als auch für die Hygiene der Mundhöhle und eine kosmetische Behandlung, welche das Natriumsalz der Hyaluronsäure mit einem mittleren Molekulargewicht zwischen 800.000 und 4.000.000 als alleinigen Wirkstoff in Kombination mit Vehikeln enthalten, unter der Bedingung, daß die genannten Zusammensetzungen keine Verbindung enthalten, die aus der Gruppe ausgewählt ist, welche aus Methylsalicylat, Natriumsalicylat, Benzylalkohol, Ölsäure, Propylenglycol, Natriumglycolat, Polyoxyethylen-10-cetylether, Natriumsalz der Ethylendiaminotetraessigsäure (EDTA), Natriumdodecylsulfat oder Dimethylsulfoxid (DMSO) besteht, wobei das genannte Verfahren das Mischen des Wirkstoffs mit den Vehikeln umfaßt.

2. Das Verfahren, wie es im Anspruch 1 beansprucht wird, dadurch gekennzeichnet, daß das mittlere Molekulargewicht der Hyaluronsäure zwischen 1.000.000 und 2.000.000 liegt.

3. Das Verfahren, wie es im Anspruch 1 beansprucht wird, dadurch gekennzeichnet, daß der Wirkstoff mit einem Gehalt im Bereich von 0,005 bis 10 Gew.%, bezogen auf das Gesamtgewicht der Zusammensetzung, vorliegt.

4. Das Verfahren, wie es im Anspruch 1 beansprucht wird, dadurch gekennzeichnet, daß die topischen Zusammensetzungen für die Therapie von entzündlichen Erkrankungen der Mundhöhle den Wirkstoff mit einem Gehalt im Bereich von 0,2 bis 10 Gew.%, bezogen auf das Gesamtgewicht der Zusammensetzung, enthalten.

5. Das Verfahren, wie es im Anspruch 4 beansprucht wird, dadurch gekennzeichnet, daß die topischen Zusammensetzungen für die Therapie von entzündlichen Erkrankungen der Mundhöhle den Wirkstoff mit einem Gehalt im Bereich von 0,2 bis 10 Gew.%, bezogen auf das Gesamtgewicht der Zusammensetzung, enthalten.

6. Das Verfahren, wie es im Anspruch 3 beansprucht wird, dadurch gekennzeichnet, daß die genannten Zusammensetzungen für die Prophylaxe von entzündlichen Erkrankungen, für die kosmetische Behandlung und Hygiene der Mundhöhle den Wirkstoff in einer Konzentration zwischen 0,005 und 0,1 Gew.%, bezogen auf das Gesamtgewicht der Zusammen-setzung, enthalten.

7. Das Verfahren, wie es im Anspruch 6 beansprucht wird, dadurch gekennzeichnet, daß die genannten Zusammensetzungen den Wirkstoff in einer Konzentration von 0,01 Gew.%, bezogen auf das Gesamtgewicht der Zusammensetzung, enthalten.

8. Das Verfahren, wie es im Anspruch 1 beansprucht wird, dadurch gekennzeichnet, daß die genannten Zusammensetzungen in der Form von Zahnfleischpasten, Zahnpasten, Mundwässern, Haftpasten und Haftpulvern vorliegen.

9. Das Verfahren, wie es in den Ansprüchen 4 oder 5 beansprucht wird, dadurch gekennzeichnet, daß die genannten Zusammensetzungen für die Behandlung von Gingivitis, Stomatitis und von Reizungen aufgrund mechanischer Ursachen oder aufgrund von Operationen verwendbar sind.

10. Das Verfahren, wie es im Anspruch 8 beansprucht wird, dadurch gekennzeichnet, daß die genannten Zahnfleischpasten während der Zahnung bei Kindern verwendbar sind.

## Revendications (Revendications pour l'(les) Etat(s) contractant(s) suivant(s): DE, FR, GB, IT)

1. Utilisation d'acide hyaluronique sous forme de son sel de sodium, caractérisé par un poids moléculaire moyen entre 800 000 et 4 000 000, comme principe actif dans la préparation de compositions pharmaceutiques pour une application topique aussi bien pour la thérapie que pour la prophylaxie d'affections inflammatoires de la cavité buccale, et pour l'hygiène et le traitement cosmétique de la cavité buccale, à condition que lesdites compositions ne contiennent aucun composé choisi dans le groupe constitué par le salicylate de méthyle, le salicylate de sodium, l'alcool benzylique, l'acide oléique, le propylène glycol, le glycolate de sodium, un polyoxyéthylène-10-cétyl éther, l'éthylènediaminetétraacétate de sodium (EDTA), le dodécylsulfate de sodium et le diméthylsulfoxyde (DMSO).

2. Utilisation d'acide hyaluronique sous la forme de son sel de sodium telle que revendiquée dans la revendication 1, caractérisée en ce que le poids moléculaire moyen de l'acide hyaluronique est entre 1 000 000 et 2 000 000.

3. Compositions topiques contenant le sel de sodium de l'acide hyaluronique comme seul principe actif conformément à l'une des revendications précédentes, en combinaison avec des excipients, pour le traitement thérapeutique et prophylactique d'affections inflammatoires de la cavité buccale, et pour l'hygiène et le traitement cosmétique de la cavité buccale, à condition que lesdites compositions ne contiennent aucun composé choisi dans le groupe constitué par le salicylate de méthyle, le salicylate de sodium, l'alcool benzylique, l'acide oléique, le propylène glycol, le glycolate de sodium, un polyoxyéthylène-10-cétyl éther, l'éthylènediaminetétraacétate de sodium (EDTA), le dodécylsulfate de sodium et le diméthylsulfoxyde (DMSO).

4. Compositions topiques selon la revendication 3, contenant le principe actif en des quantités allant de 0,005 à 10% en poids par rapport au poids total de la composition.

5. Compositions topiques pour le traitement thérapeutique d'affections inflammatoires de la cavité buccale selon la revendication 4, contenant le principe actif en des quantités allant de 0,2 à 10% en poids par rapport au poids total de la composition.

6. Compositions topiques pour le traitement thérapeutique d'affections inflammatoires de la cavité buccale selon la revendication 5, contenant le principe actif en des quantités allant de 0,2 à 1% en poids par rapport au poids total de la composition.

7. Compositions topiques pour la prophylaxie, le traitement cosmétique et l'hygiène de la cavité buccale selon la revendication 4, contenant le principe actif en une concentration entre 0,005 et 0,1% en poids par rapport au poids total de la composition.

8. Compositions topiques selon la revendication 7, contenant le principe actif en une concentration de 0,01% en poids par rapport au poids total de la composition.

9. Compositions selon la revendication 3, sous forme de pâtes gingivales, de pâtes dentifrices, de liquides pour bains de bouche, de pâtes et de poudres adhésives.

10. Compositions topiques selon l'une des revendications 5 et 6 pour le traitement de gingivites, de stomatites et d'irritations dues à des causes mécaniques ou chirurgicales.

11. Pâtes gingivales selon la revendication 9, pour les poussées dentaires chez l'enfant.

## Revendications (Revendications pour l'(les) Etat(s) contractant(s) suivant(s): ES, GR)

1. Procédé pour la préparation de compositions pour une application topique aussi bien pour la thérapie que pour la prophylaxie d'affections inflammatoires de la cavité buccale, et pour l'hygiène et le traitement cosmétique de la cavité buccale, contenant le sel de sodium de l'acide hyaluronique ayant un poids moléculaire moyen entre 800 000 et 4 000 000, comme seul principe actif, en combinaison avec des excipients, à condition que lesdites compositions ne contiennent aucun composé choisi dans le groupe constitué par le salicylate de méthyle, le salicylate de sodium, l'alcool benzylique, l'acide oléique, le propylène glycol, le glycolate de sodium, un polyoxyéthylène-10-cétyl éther, l'éthylènediaminetétraacétate de sodium (EDTA), le dodécylsulfate de sodium et le diméthylsulfoxyde (DMSO), ledit procédé comprenant l'opération de mélange du principe actif avec les excipients.

2. Procédé selon la revendication 1, caractérisé en ce que le poids moléculaire moyen de l'acide hyaluronique est entre 1 000 000 et 2 000 000.

3. Procédé selon la revendication 1, caractérisé en ce que le principe actif est en des quantités allant de 0,005 à 10% en poids par rapport au poids total de la composition.

4. Procédé selon la revendication 1, caractérisé en ce que les compositions pour le traitement thérapeutique d'affections inflammatoires de la cavité buccale contiennent le principe actif en des quantités allant de 0,2 à 10% en poids par rapport au poids total de la composition.

5. Procédé selon la revendication 4, caractérisé en ce que les compositions pour le traitement thérapeutique d'affections inflammatoires de la cavité buccale contiennent le principe actif en des quantités allant de 0,2 à 1% en poids par rapport au poids total de la composition.

6. Procédé selon la revendication 3, caractérisé en ce que lesdites compositions pour la prophylaxie d'affections inflammatoires, le traitement cosmétique et l'hygiène de la cavité buccale contiennent le principe actif en une concentration entre 0,005 et 0,1% en poids par rapport au poids total de la composition.

7. Procédé selon la revendication 6, caractérisé en ce que lesdites compositions contiennent le principe actif en une concentration de 0,01% en poids par rapport au poids total de la composition.

8. Procédé selon la revendication 1, caractérisé en ce que les compositions sont sous forme de pâtes gingivales, de pâtes dentifrices, de liquides pour bains de bouche, de pâtes et de poudres adhésives.

9. Procédé selon l'une des revendications 4 ou 5, caractérisé en ce que lesdites compositions sont utiles pour le traitement de gingivites, de stomatites et d'irritations dues à des causes mécaniques ou chirurgicales.

10. Procédé selon la revendication 8, caractérisé en ce que lesdites pâtes gingivales sont utiles durant les poussées dentaires chez l'enfant.
